(19) Europäisches Patentamt
European Patent Office
Office européen des brevets

(11) Veröffentlichungsnummer: **0 042 107**
**B1**

(12) # EUROPÄISCHE PATENTSCHRIFT

(45) Veröffentlichungstag der Patentschrift: **03.04.85**

(51) Int. Cl.⁴: **C 07 D 237/04,** A 61 K 31/50

(21) Anmeldenummer: **81104267.0**

(22) Anmeldetag: **03.06.81**

(54) **Neue Dihydropyridazinone, Verfahren zu ihrer Herstellung und diese Verbindungen enthaltende therapeutische Mittel.**

(30) Priorität: **13.06.80 DE 3022176**

(43) Veröffentlichungstag der Anmeldung:
**23.12.81 Patentblatt 81/51**

(45) Bekanntmachung des Hinweises auf die Patenterteilung:
**03.04.85 Patentblatt 85/14**

(84) Benannte Vertragsstaaten:
**AT BE CH DE FR GB IT LI LU NL SE**

(56) Entgegenhaltungen:
**US-A-3 689 652**
**US-A-4 011 321**

(73) Patentinhaber: **BASF Aktiengesellschaft**
**Carl-Bosch-Strasse 38**
**D-6700 Ludwigshafen (DE)**

(72) Erfinder: **Thyes, Marco, Dr.**
**Thorwaldsenstrasse 1**
**D-6700 Ludwigshafen (DE)**
Erfinder: **Koenig, Horst, Dr.**
**Pariser Strasse 4**
**D-6700 Ludwigshafen (DE)**
Erfinder: **Lenke, Dieter, Dr.**
**Kekuleplatz 1**
**D-6700 Ludwigshafen (DE)**
Erfinder: **Lehmann, Hans Dieter, Dr.**
**Im Hefen 15**
**D-6945 Hirschberg-Leutershausen (DE)**
Erfinder: **Gries, Josef, Dr.**
**Roemerweg 43**
**D-6706 Wachenheim (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 042 107 B1

**Beschreibung**

Die Erfindung betrifft neue 6-(p-Acylaminophenyl)-4,5-dihydro-3(2H)-pyridazinone, deren Acylrest sich von einer Cycloalkylcarbonsäure ableitet, Verfahren zur ihrer Herstellung, diese Verbindungen enthaltende pharmazeutische Zubereitungen und ihre Verwendung als Arzneimittel bei thrombo-embolischen Erkrankungen und als Antihypertensiva.

6-(Acylamino)phenyl-4,5-dihydro-3(2H)-pyridazinone sind bereits mehrfach beschreiben worden, beispielsweise werden in der DE—OS 16 70 158 in den Stellungen 4 und 5 unsubstituierte 6-(Acylamino)phenyl-4,5-dihydro-3(2H)-pyridazinone mit blutdrucksenkenden und entzündungshemmenden Eigenschaften beschrieben. Für den in der Acylaminogruppe dieser Dihydropyridazinone enthaltenen Acylrest sind beispielsweise Alkanoyl und Cycloalkylcarbonyl mit 5 bis 7 C—Atomen im Ring angegeben. 6-(p-Aminoalkanoylaminophenyl)-4,5-dihydro-3(2H)-pyridazinone, in 4- und 5-Stellung unsubstituiert, sind gemäß der DE—OS 21 23 246 blutdrucksenkend, coronarerweiternd und antiinflammatorisch wirksam. Für 6-(p-Acylaminophenyl)-4,5-dihydro-3(2H)-pyridazinone, die in 4-Stellung eine Alkylgruppe tragen, werden in der DE—OS 23 04 977 cardiovasculäre und antiphlogistische Eigenschaften genannt. In der DE—OS 21 50 436 und den US—PS 3 824 271 und 3 888 901 werden blutdrucksenkend wirkende, in 5-Stellung durch einen Alkylrest substituierte 6-Alkanoylaminophenyl-4,5-dihydro-3(2H)-pyridazinone angegeben. Aus einer Veröffentlichung von F.J. McEvoy und G.R. Allen, Jr. (J.Med.Chem. *17,* 281 ff. (1974) geht das blutdrucksenkend wirkende 4,5-Dihydro-5-methyl-6-(p-trifluoracetylaminophenyl)-3(2H)-pyridazinon hervor. Schließlich sind auch 6-(p-Alkanoylaminophenyl)-4,5-dihydro-3(2H)-pyridazinone, die im Alkanoylrest durch ein Halogenatom oder mehrere Halogenatome und gegebenenfalls in der 5-Stellung durch einen Alkylrest substituiert sind, als Arzneimittel wegen ihrer thrombozytenaggregationshemmenden und blutdrucksenkenden Eigenschaften vorgeschlagen worden DE—OS 27 27 481 und DE—OS 28 54 191).

Es wurde nun gefunden, daß 6-(p-Acylaminophenyl)-4,5-dihydro-3(2H)-pyridazinone der allgemeinen Formel I

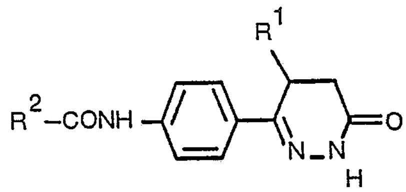

I,

in der $R^1$ für ein Wasserstoffatom oder Methyl steht und $R^2$ einen Cycloalkylrest mit 3 oder 4 C—Atomen im Ring, der gegebenenfalls ein- bis dreifach durch Halogenatome und/oder Methylreste substituiert ist, darstellt, wertvolle pharmakologische Eigenschaften aufweisen.

Als unsubstituierte bzw. ein- bis dreifach durch Halogenatome, wie Chlor, Brom und Fluor, und/oder Methylreste substituierte Cycloalkylrest für $R^2$ seien Cyclopropyl, 1-Methylcyclopropyl, 2-Methylcyclopropyl, 2,2-Dimethylcyclopropyl, 1,2,2-Trimethylcyclopropyl, 2,2,3-Trimethylcyclopropyl, 1-Chlorcyclopropyl, 2-Bromcyclopropyl, 2,2-Dichlorcyclopropyl, 2,2-Dibromcyclopropyl, 2,2-Dichlor-1-methylcyclopropyl, Cyclobutyl, 1-Methylcyclobutyl, 2-Methylcyclobutyl, 3-Methylcyclobutyl, 3,3-Dimethylcyclobutyl, 1-Chlorcyclobutyl, 2-Chlorcyclobutyl, 3-Chlorcyclobutyl, 1-Bromcyclobutyl, 3-Bromcyclobutyl, 3-Chlor-3-methylcyclobutyl und 1-Brom-3,3-dimethylcyclobutyl genannt.

Besonders bevorzugt im Hinblick auf die Wirkung sind Verbindungen, in denen $R^2$ einen gegebenenfalls einbis dreifach durch Halogenatome, insbesondere Chlor- oder Bromatome, und/oder Methylreste substituierten Cyclopropylrest bedeutet.

Die Dihydropyridazinone der Formel I können hergestellt werden, indem man eine Verbindung der Formel II

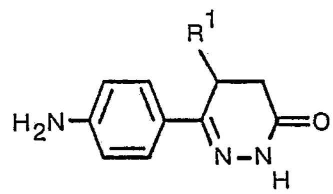

II,

in der $R^1$ die für Formel I angegebenen Bedeutungen hat, mit einem Acylierungsmittel der Formel III

$$R^2COY \qquad III,$$

in der $R^2$ die für Formel I angegebenen Bedeutungen hat und für Y OH, ein Halogenatom, insbesondere Chlor, ein niederer Alkoxyrest oder $OCOR^2$ steht, in an sich bekannter Weise umsetzt.

Gemäß den für Y angegebenen Bedeutungen sind zweckmäßige Acylierungsmittel die entsprechenden Carbonsäuren, Carbonsäurehalogenide, insbesondere Chloride, Carbonsäureester, insbesondere Methyl- und Ethylester, und die entsprechenden Carbonsäureanhydride.

Die Acylierung wird unter an sich üblichen Bedingungen durchgeführt. In der Regel unter Verwendung von wenigstens einer äquimolaren Menge des Acylierungsmittels, zweckmäßig in Gegenwart eines Lösungsmittels und gegebenenfalls in Gegenwart einer Hilfsbase als säurebindendes Mittel bei Temperaturen zwischen 0 und 160°C, gegebenenfalls bei den Siedetemperaturen des Reaktionsgemisches und gegebenenfalls unter Anwendung von Druck.

Als Lösungsmittel kommen unter den Reaktionsbedingungen inerte Lösungsmittel, wie aromatische Kohlenwasserstoffe, beispielsweise Benzol oder Toluol, cyclische aliphatische Ether, wie Tetrahydrofuran oder Dioxan, oder Dialkylformamide, wie Dimethylformamid, in Betracht. Hilfsbasen als säurebindende Mittel sind zweckmäßigerweise anorganische Basen, wie Natrium- oder Kaliumcarbonat, Natrium- oder Kalium-hydrogencarbonat, oder tertiäre organische Amine, wie Triäthylamin.

Gemäß einer weiteren Ausführungsform werden die neuen Verbindungen der Formel I erhalten, indem man eine Acylaminoverbindung der Formel IV

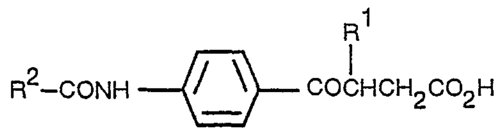

IV,

in der $R^1$ und $R^2$ die für Formel I angegebenen Bedeutungen haben, mit Hydrazin cyclisiert.

Diese Ringschlußreaktion mit Hydrazin, das bevorzugt als Hydrat eingesetzt wird, erfolgt vorteilhaft in einem unter den Reaktionsbedingungen inerten Lösungsmittel, insbesondere einem niederen Alkohol, wie Methanol, Ethanol oder Propanol, einem cyclischen aliphatischen Ether, wie Tetrahydrofuran oder Dioxan, oder einem Dialkylformamid, wie Dimethylformamid, und bei Temperaturen von 60 bis 150°C, vorzugsweise 80 bis 120°C. In der Regel werden hierbei pro Mol einer Verbindung der Formel IV 1 bis 1,2 Mol Hydrazin oder Hydrazinhydrat verwendet.

Die Ausgangsverbindungen der Formel IV können hergestellt werden, indem man eine Aminosäure der Formel V

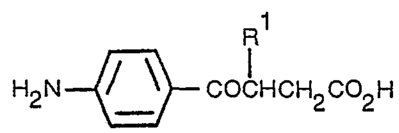

V,

in der $R^1$ die für Formel I angegebenen Bedeutungen hat, mit einem Acylierungsmittel der Formel III

$$R^2COY \qquad III,$$

in der $R^2$ und Y die oben angegebenen Bedeutungen haben, unter den oben angegebenen Bedingungen acyliert.

Zu den Verbindungen der Formel IV, in denen $R^1$ Wasserstoff ist und $R^2$ die entsprechenden oben angegebenen Bedeutungen hat, kann man auch gelangen, indem man ein Anilid der Formel VI

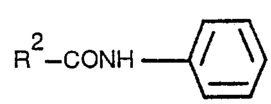

VI,

in der $R^2$ die angegebenen Bedeutungen hat, mit Bernsteinsäureanhydrid in Gegenwart von Aluminiumchlorid unter den Bedingungen einer Friedel-Crafts-Acylierung umsetzt.

Diese Acylierung nach Friedel-Crafts kann in einem Lösungsmittel, beispielsweise Schwefelkohlenstoff, bei Temperaturen von 0 bis 60°C durchgeführt werden. Sie kann auch in einer Dimethylformamid/Aluminiumchlorid-Schmelze bei Temperaturen zwischen 50 und 120°C, vorzugsweise 60 bis 90°C, erfolgen. Dabei ist es zweckmäßig auf 1 Mol Bernsteinsäureanhydrid bzw. 1 Mol Anilid der Formel VI etwa 10 Mol Aluminiumchlorid und etwa 2,5 Mol Dimethylformamid zu verwenden.

Ferner können die Verbindungen der Formel IV, in denen $R^1$ für Methyl steht und $R^2$ die oben angegebenen Bedeutungen hat, hergestellt werden, indem man ein Anilid der Formel VI, in der $R^2$ die angegebenen Bedeutungen hat, mit Citraconsäureanhydrid in Gegenwart von Aluminiumchlorid unter den Bedingungen einer Friedel-Crafts-Reaktion umsetzt und die erhaltene 3-(p-Acylaminobenzoyl)crotonsäure der Formel VII

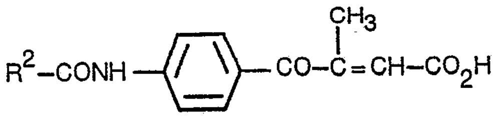

VII

mit Zink in Eisessig behandelt. Hierzu kann beispielsweise unter den Bedingungen gearbeitet werden, die in der DE—OS 21 50 436 für die Herstellung einer 3-(p-Alkanoylaminobenzoyl)buttersäure der Formel VIII

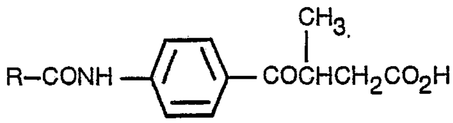

VIII R = Alkyl

aus einem Anilid der Formel IX

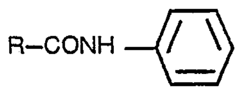

IX R = Alkyl

angegeben werden.

Dihydropyridazinone der Formel I, in denen $R^2$ einen durch Halogen substituierten Cycloalkylrest bedeutet, werden bevorzugt über die Umsetzung einer Verbindung der Formel II mit einem Acylierungsmittel der Formel III oder über die Cyclisierung einer Acylaminoverbindung der Formel IV mit Hydrazin hergestellt.

Die als Ausgangssubstanzen verwendeten Verbindungen der Formel II und auch der Formel V sind bekannt oder können beispielsweise unter den in den DE—OS 16 70 158 und 21 50 436 oder den US—PS 3 824 271 und 3 888 901 beschriebenen Bedingungen hergestellt werden.

Erfindungsgemäße Verbindungen, die nach den genannten Verfahren erhalten werden, sind beispielsweise:

6-(p-Cyclopropylcarbonylaminophenyl)-4,5-dihydro-3(2H)-pyridazinon;
6-(p-Cyclopropylcarbonylaminophenyl)-4,5-dihydro-5-methyl-3(2H)-pyridazinon;
4,5-Dihydro-6-[p-(1-methylcyclopropylcarbonylamino)phenyl]-3(2H)-pyridazinon;
4,5-Dihydro-5-methyl-6-[p-(1-methylcyclopropylcarbonylamino)phenyl]-3(2H)-pyridazinon;
cis- und trans-4,5-Dihydro-6-[p-(2-methylcyclopropylcarbonylamino)phenyl]-3(2H)-pyridazinon;
cis- und trans-4,5-Dihydro-5-methyl-6-[p-(2-methylcyclopropylcarbonylamino)phenyl]-3(2H)-pyridazinon;
4,5-Dihydro-6-[p-(2,2-dimethylcyclopropylcarbonylamino)phenyl]-3(2H)-pyridazinon;
4,5-Dihydro-6-[p-(2,2-dimethylcyclopropylcarbonylamino)phenyl]-5-methyl-3(2H)-pyridazinon;
cis- und trans-4,5-Dihydro-5-methyl-6-[p-(2,2,3-trimethylcyclopropylcarbonylamino)phenyl]-3(2H)-pyridazinon;
6-[p-(1-Chlorcyclopropylcarbonylamino)phenyl]-4,5-dihydro-3(2H)-pyridazinon;
6-[p-(1-Chlorcyclopropylcarbonylamino)phenyl]-4,5-dihydro-5-methyl-3(2H)-pyridazinon;
cis- und trans-6-[p-(2-Bromcyclopropylcarbonylamino)phenyl]-4,5-dihydro-3(2H)-pyridazinon;
cis- und trans-6-[p-(2-Bromcyclopropylcarbonylamino)phenyl]-4,5-dihydro-5-methyl-3(2H)-pyridazinon;
6-[p-(2,2-Dichlorcyclopropylcarbonylamino)phenyl]-4,5-dihydro-3(2H)-pyridazinon;
6-[p-(2,2-Dichlorcyclopropylcarbonylamino)phenyl]-4,5-dihydro-5-methyl-3(2H)-pyridazinon;
6-[p-(2,2-Dibromcyclopropylcarbonylamino)phenyl]-4,5-dihydro-5-methyl-3(2H)-pyridazinon;
6-[p-(2,2-Dichlor-1-methylcyclopropylcarbonylamino)phenyl]-4,5-dihydro-3(2H)-pyridazinon;
6-[p-(2,2-Dichlor-1-methylcyclopropylcarbonylamino)phenyl]-4,5-dihydro-5-methyl-3(2H)-pyridazinon;
6-(p-Cyclobutylcarbonylaminophenyl)-4,5-dihydro-3(2H)-pyridazinon;
6-(p-Cyclobutylcarbonylaminophenyl)-4,5-dihydro-5-methyl-3(2H)-pyridazinon;
4,5-Dihydro-5-methyl-6-[p-(1-methylcyclobutylcarbonylamino)phenyl]-3(2H)-pyridazinon;
cis- und trans-4,5-Dihydro-5-methyl-6-[p-(2-methylcyclobutylcarbonylamino)phenyl]-3(2H)-pyridazinon;
cis- und trans-4,5-Dihydro-5-methyl-6-[p-(3-methylcyclobutylcarbonylamino)phenyl]-3(2H)-pyridazinon;
6-[p-(1-Chlorcyclobutylcarbonylamino)phenyl]-4,5-dihydro-3(2H)-pyridazinon;
6-[p-(1-Chlorcyclobutylcarbonylamino)phenyl]-4,5-dihydro-5-methyl-3(2H)-pyridazinon;
cis- und trans-6-[p-(2-Chlorcyclobutylcarbonylamino)phenyl]-4,5-dihydro-5-methyl-3(2H)-pyridazinon;
cis- und trans-6-[p-(3-Chlorcyclobutylcarbonylamino)phenyl]-4,5-dihydro-3(2H)-pyridazinon;
cis- und trans-6-[p-(3-Chlorcyclobutylcarbonylamino)phenyl]-4,5-dihydro-5-methyl-3(2H)-pyridazinon;
6-[p-(1-Bromcyclobutylcarbonylamino)phenyl]-4,5-dihydro-5-methyl-3(2H)-pyridazinon;
6-[p-(1-Brom-3,3-dimethylcyclobutylcarbonylamino)phenyl]-4,5-dihydro-5-methyl-3(2H)-pyridazinon.

Es wird darauf hingewiesen, daß die Verbindungen der Formel I, in denen $R^1$ Methyl ist, ein

asymmetrisches Kohlenstoffatom in 5-Stellung aufweisen und als Racemate erhalten werden. Die vorliegende Erfindung soll die Enantiomeren mit einschließen. Ist eine Trennung erwünscht, so wird diese vorteilhaft auf der Stufe einer Verbindung der Formel II nach bekannten Methoden, z.B. Bildung diastereomerer Salze mit optisch aktiven Hilfssäuren, wie Dibenzoylweinsäure oder Campher-10-sulfonsäure, durchgeführt.

Bei geeignetem Rest $R^2$, z.B. 2-Methylcyclopropyl, tritt ferner in den Verbindungen der Formel I eine geometrische cis-trans-Isomerie auf. Die vorliegende Erfindung betrifft die jeweiligen cis- und trans-Isomeren und deren Gemische. Die reinen cis- oder trans-Verbindungen werden erhalten, indem man reine Ausgangsverbindungen verwendet, oder können gegebenenfalls durch Kristallisation getrennt werden.

Die erfindungsgemäßen Dihydropyridazinone der Formel I zeichnen sich durch thrombozytenaggregationshemmende und blutdrucksenkende Eigenschaften aus. Sie sind als Antihypertensiva und zur Prophylaxe und Therapie thrombo-embolischer Erkrankungen geeignet.

Die vorteilhafte thrombozytenaggregationshemmende Wirkung kann im Vergleich zu Acetylsalicylsäure z.B. an der durch Collagen induzierten Aggregation von Thrombozyten des Menschen festgestellt werden. Zum Nachweis der blutdrucksenkenden Wirkung werden z.B. Ratten in Urethan-Narkose verwendet. Als Referenzsubstanz kann hier z.B. Dihydralazin verwendet werden.

Im einzelnen wurden zur Untersuchung der pharmakodynamischen Eigenschaften folgende Methoden verwendet:

1. Hemmung der durch Collagen induzierten Aggregation von Thrombozyten der Ratte ex vivo.

Die Substanzen werden Gruppen von 10 bis 15 männlichen Sprague-Dawley-Ratten (200 bis 250 g) oral appliziert. 1 Stunde nach der Applikation wird in Äther-Narkose Blut entnommen und durch Zentrifugation (300 g, 10 min Dauer bei 4°C) thrombozytenreiches Plasma gewonnen. Die photometrische Messung der Thrombozytenaggregation erfolgt unter Zusatz von Magnesiumchlorid (Endkonzentration 100 mmol/1) und von Collagen Stago (Endkonzentration 0,02 mg/ml) im Born-Aggregometer Mk 3. Als Aggregationsmaß findet die maximale Extinktionsänderung/sec Verwendung.

Als ED 33% wird die Dosis bestimmt, welche die durch Collagen induzierte Thrombozytenaggregation um 33% hemmt.

2. Antihypertensive Wirkung an der spontan hypertonen Ratte

Die Substanzen werden männlichen spontan hypertonen Okamoto-Ratten (4 bis 8 Tiere/Dosis, Gewicht: 270 bis 360 g) oral appliziert. Der systolische Blutdruck wird vor und 2 Stunden nach der Applikation unblutig am Rattenschwanz mit Hilfe von Piezokristallaufnehmern ermittelt.

Als ED 20% wird unter Berücksichtigung der Werte unbehandelter Kontrolltiere die Dosis bestimmt, welche den systolischen Druck um 20% senkt.

Die erhaltenen Ergebnisse sind in der folgenden Tabelle 1 zusammengestellt, wobei zur Erläuterung ausgeführt wird:

Als Vergleichssubstanzen mit der relativen Wirksamkeit (R.W.) von 1 dienen die anerkannten Mittel Acetylsalicylsäure (ASS) und Dihydralazin. Weiterhin wurde verglichen mit einer strukturell ähnlichen Verbindung aus dem Stand der Technik, 6-[p-(2-Chlorpropionylamino)-phenyl]-4,5-dihydro-5-methyl-3(2H)-pyridazinon (Verbindung $V_1$), die sich bereits als recht wirksam erwiesen hat.

Die gefundenen Zahlenwerte zeigen, daß alle in der Tabelle aufgeführten erfindungsgemäßen Verbindungen im Hinblick auf die sich im Handel befindlichen Verbindungen wenigstens in einer der untersuchten Wirkungen eine wesentliche höhere Wirkung aufweisen.

TABELLE 1

| Verbindung Bsp. Nr. | Thrombozytenaggregations-Hemmung ED 33% | R.W. | Antihypertensive Wirkung ED 20% | R.W. |
|---|---|---|---|---|
| 1, 2 | 0,357 | 652,66 | 1,79 | 3,83 |
| 3 | 0,240 | 970,83 | 0,155 | 44,199 |
| 14 | 16,9 | 13,79 | 0,681 | 10,06 |
| 15 | 2,00 | 116,5 | 0,168 | 40,77 |
| 16 | >10,0 | <23,30 | 2,67 | 2,57 |
| $V_1$ | 0,82 | 284,15 | 1,16 | 5,91 |
| ASS | 233 | 1,00 | – | – |
| Dihydralazin | – | – | 6,85 | 1,00 |

Gegenstand der vorliegenden Erfindung sind demnach auch therapeutische Mittel oder Zubereitungen, die neben pharmazeutisch üblichen Träger- und Verdünnungsmitteln eine Verbindung der Formel I als Wirkstoff enthalten, sowie die Verwendung dieser Verbindungen bei der Behandlung von hohem Blutdruck und thrombo-embolischen Erkrankungen.

Die therapeutischen Mittel oder Zubereitungen werden mit den üblichen Trägerstoffen oder Verdünnungsmitteln und den üblicherweise verwendeten pharmazeutisch technischen Hilfsstoffen entsprechend der gewünschten Applikationsart mit einer geeigneten Dosierung in bekannter Weise hergestellt. Dabei kommen beim Menschen Dosen von 1 bis 100 mg, bevorzugt 5 bis 50 mg, in Betracht, wobei die orale Applikation bevorzugt ist.

Darreichungsformen, die zur oralen Applikation geeignet sind, sind beispielsweise Tabletten, Filmtabletten, Dragees, Kapseln, Pillen, Pulver, Lösungen, Suspensionen oder Depotformen.

Zur praktischen Anwendung werden die erfindungsgemäß zu verwendenden Verbindungen mit den in der galenischen Pharmazie üblichen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Maisstärke, Alginsäure oder Polyvinylpyrrolidon, Bindemitteln, wie Stärke oder Gelatine, Gleitmitteln, wie Magnesiumstearat oder Talkum, und/oder Mitteln zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen (vgl. L.G. Godman, A. Gilman, The Pharmacological Basis of Therapeutics).

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Die Herstellung der neuen 6-(p-Acylaminophenyl)-4,5-dihydro-3(2H)-pyridazinone wird durch die folgenden Beispiele näher erläutert.

### Beispiel 1

6,0 g (31,7 mmol) 6-(p-Aminophenyl)-4,5-dihydro-3(2H)-pyridazinon werden mit 4,0 g (38,3 mmol) Cyclopropancarbonsäurechlorid in 100 ml absolutem Toluol 6 Stunden bei 80°C gehalten. Man saugt bei 10°C ab, wäscht zuerst mit Toluol, dann mit Wasser und kristallisiert aus Dimethylformamid/Wasser um. Man erhält 5,0 g (61% d.Th.) 6-(p-Cyclopropylcarbonylaminophenyl)-4,5-dihydro-3(2H)-pyridazinon, farblose Kristalle, Schmelzpunkt 272 bis 273°C.

Analyse für $C_{14}H_{15}N_3O_2$:
ber.: C 65,4 H 5,9 N 16,3%
gef.: C 65,0 H 5,9 N 16,5%

### Beispiel 2

a) Zu einer Lösung von 5,0 g (25,9 mmol) 3-(p-Aminobenzoyl)propionsäure in 100 ml absolutem Tetrahydrofuran gibt man unter Rühren bei Raumtemperatur tropfenweise 3,2 g (30,6 mmol) Cyclopropancarbonsäurechlorid, gelöst in 20 ml absolutem Tetrahydrofuran, wobei sich ein Niederschlag bildet. Anschließend wird das Reaktionsgemisch 7 Stunden am Rückfluß gehalten. Man saugt bei 10°C ab, wäscht mit Wasser und trocknet unter vermindertem Druck bei 50°C. Man erhält 5,1 g (75% d.Th.) 3-(p-Cyclopropylcarbonylaminobenzoyl)propionsäure, gelbe Kristalle, Schmelzpunkt 229 bis 230°C (umkristallisiert aus Ethanol/Wasser).

Analyse für $C_{14}H_{15}NO_4$:
ber.: C 64,4 H 5,8 N 5,4%
gef.: C 64,5 H 5,9 N 5,3%

b) 2,0 g (7,7 mmol) 3-(p-Cyclopropylcarbonylaminobenzoyl)propionsäure werden mit 0.45 g (9,0 mmol) Hydrazinhydrat und 20 ml Ethanol 6 Stunden am Rückfluß gehalten. Nach dem Absaugen bei 10°C und dem Umkristallisieren aus Dimethylformamid/Wasser isoliert man 1,35 g (68% d.Th.) 6-(p-Cyclopropylcarbonylaminophenyl)-4,5-dihydro-3(2H)-pyridazinon, farblose Kristalle (identisch mit der Verbindung aus Beispiel 1).

### Beispiel 3

6,0 g (29,5 mmol) 6-(p-Aminophenyl)-4,5-dihydro-5-methyl-3(2H)-pyridazinon werden mit 3,4 g (32,5 mmol) Cyclopropancarbonsäurechlorid in 100 ml absolutem Toluol 6 Stunden bei 80°C gehalten. Man saugt bei 10°C ab, wäscht zuerst mit Toluol, dann mit Wasser und kristallisiert aus Dimethylformamid/Wasser um. Man isoliert 6,8 g (85% d.Th.) 6-(p-Cyclopropylcarbonylaminophenyl)-4,5-dihydro-5-methyl-3(2H)-pyridazinon, beige Kristalle, Schmelzpunkt 267 bis 268°C.

Analyse für $C_{15}H_{17}N_3O_2$:
ber.: C 66,4 H 6,3 N 15,5%
gef.: C 66,3 H 6,5 N 15,7%

Beispiel 4

6,0 g (29,5 mmol) 6-(p-Aminophenyl)-4,5-dihydro-5-methyl-3(2H)-pyridazinon werden unter Rühren in 150 ml absolutem Tetrahydrofuran durch Erwärmen auf 60°C gelöst. Zu dieser Lösung tropft man — bei 60°C — 4,2 g (35,4 mmol) 1-Methylcyclopropancarbonsäurechlorid und rührt das Reaktionsgemisch noch 7 Stunden bei Rückflußtemperatur nach. Man saugt bei 10°C ab, wäscht mit Wasser und kristallisiert zweimal aus Dimethylformamid/Wasser um. Man isoliert 5,5 g (65% d.Th.) 4,5-Dihydro-5-methyl-6-[p-(1-methylcyclopropylcarbonylamino)phenyl]-3(2H)-pyridazinon, fast farblose Kristalle, Schmelzpunkt 235 bis 237°C.

Analyse für $C_{16}H_{19}N_3O_2$:
ber.: C 67,3 H 6,7 N 14,7%
gef.: C 67,2 H 6,7 N 14,9%

Beispiel 5

5,0 g (26,4 mmol) 6-(p-Aminophenyl)-4,5-dihydro-3(2H)-pyridazinon werden mit 4,2 g (31,7 mmol) 2,2-Dimethylcyclopropancarbonsäurechlorid in 100 ml absolutem Tetrahydrofuran 7 Stunden am Rückfluß gehalten. Man engt auf circa 50 ml ein, saugt bei 10°C ab, wäscht mit Wasser und kristallisiert aus Dimethylformamid/Wasser um. Man isoliert 5,2 g (68% d.Th.) 4,5-Dihydro-6-[p-(2,2-dimethylcyclopropylcarbonylamino)phenyl]-3(2H)-pyridazinon-viertelhydrat, fast farblose Kristalle, Schmelpunkt 213 bis 214°C.

Analyse für $C_{16}H_{19}N_3O_2$. 1/4 $H_2O$:
ber.: C 66,3 H 6,8 N 14,5%
gef.: C 66,5 H 6,8 N 14,3%

Beispiel 6

Analog Beispiel 4 werden 6,0 g (29,5 mmol) 6-(p-Aminophenyl)-4,5-dihydro-5-methyl-3(2H)-pyridazinon mit 4,7 g (35,4 mmol) 2,2-Dimethylcyclopropancarbonsäurechlorid in 150 ml absolutem Tetrahydrofuran umgesetzt. Man engt auf ca. 70 ml ein, gibt Wasser zu und saugt ab. Nach dem Waschen mit Wasser und zwei Umkristallisationen aus Dimethylformamid/Wasser erhält man 5,8 g (66% d.Th.) 4,5-Dihydro-6-[p-(2,2-dimethylcyclopropylcarbonylamino)-phenyl]-5-methyl-3(2H)-pyridazinon, farblose Kristalle, Schmelzpunkt 222 bis 223°C.

Analyse für $C_{17}H_{21}N_3O_2$:
ber.: C 68,2 H 7,1 N 14,0%
gef.: C 68,2 H 7,4 N 13,7%

Beispiel 7

Zu einer Suspension von 5,0 g (26,4 mmol) 6-(p-Aminophenyl)-4,5-dihydro-3(2H)-pyridazinon in 100 ml absolutem Tetrahydrofuran tropft man unter Rühren eine Lösung von 4,45 g (32,0 mmol) 1-Chlorcyclopropancarbonsäurechlorid in 20 ml absolutem Tetrahydrofuran. Anschließend wird zuerst 20 Stunden bei Raumtemperatur und dann noch 1 Stunde bei Rückflußtemperatur nachgerührt. Man saugt bei 10°C ab, wäscht zuerst mit Tetrahydrofuran, dann mit Wasser und kristallisiert aus Dimethylformamid/Wasser um. Man isoliert 6,3 g (82% d.Th.) 6-[p-(1-Chlorcyclopropylcarbonylamino)phenyl]-4,5-dihydro-3(2H)-pyridazinon, farblose Kristalle, Schmelzpunkt 231 bis 232°C.

Analyse für $C_{14}H_{14}ClN_3O_2$:
ber.: C 57,6 H 4,8 Cl 12,2 N 14,4%
gef.: C 57,8 H 5,0 Cl 11,8 N 14,7%

Beispiel 8

a) Zu 4,5 g (23,3 mmol) 3-(p-Aminobenzyl)-propionsäure in 100 ml absolutem Tetrahydrofuran gibt man unter Rühren 3,9 g (28,1 mmol) 1-Chlorcyclopropancarbonsäurechlorid. Anschließend wird das Reaktionsgemisch zuerst 20 Stunden bei Raumtemperatur und dann noch 7 Stunden bei Rückflußtemperatur nachgerührt. Man filtriert von einer geringen Menge Festsubstanz, die verworfen wird, ab und engt ein. Nach dem Umkristallisieren des Rückstandes aus Essigester isoliert man 3,4 g (49% d.Th.) 3-[p-(1-Chlorcyclopropylcarbonylamino)benzoyl]propionsäure, farblose Kristalle, Schmelzpunkt 178 bis 179°C.

Analyse für $C_{14}H_{14}ClNO_4$:
ber.: C 56,9 H 4,8 Cl 12,0 N 4,7%
gef.: 57,0 H 4,6 Cl 11,6 N 4,5%

b) 2,75 g (9,3mmol) 3-[p-(1-Chlorcyclopropylcarbonylamino)benzoyl]propionsäure werden mit 0,47 g (9,4 mmol) Hydrazinhydrat und 20 ml Ethanol 6 Stunden am Rückfluß gehalten. Nach dem Absaugen bei 10°C, dem Waschen mit Ethanol und dem Trocknen unter vermindertem Druck bei 50°C isoliert man 2,5 g (92% d.Th.) 6-[p-(1-Chlorcyclopropylcarbonylamino)phenyl]-4,5-dihydro-3(2H)-pyridazinon, farblose Kristalle (identisch mit der Verbindung aus Beispiel 7).

Beispiel 9

5,0 g (24,6 mmol) 6-(p-Aminophenyl)-4,5-dihydro-5-methyl-3(2H)-pyridazinon werden unter Rühren in 100 ml absolutem Tetrahydrofuran durch Erwärmen auf 60°C gelöst. Man läßt die Lösung auf Raumtemperatur abkühlen, tropft 4,15 g (29,9 mmol) 1-Chlorcyclopropancarbonsäurechlorid zu und rührt noch 6

Stunden bei Raumtemperatur nach. Die Reaktionslösung wird zur Hälfte eingeengt und anschließend mit 200 ml Wasser versetzt. Der ausgefallene Niederschlag wird abgesaugt und mit Wasser gewaschen. Nach dem Umkristallisieren aus Methanol erhält man 5,5 g (73% d.Th.) 6-[p-(1-Chlorcyclopropylcarbonylamino)phenyl]-4,5-dihydro-5-methyl-3(2H)-pyridazinon, farblose Kristalle, Schmelzpunkt 189 bis 190°C.

Analyse für $C_{15}H_{16}ClN_3O_2$:
ber.: C 58,9 H 5,3 Cl 11,6 N 13,7%
gef.: C 59,0 H 5,3 Cl 11,9 N 13,8%

Beispiel 10

6,0 g (31,7 mmol) 6-(p-Aminophenyl)-4,5-dihydro-3(2H)-pyridazinon werden mit 6,6 g (38,1 mmol) 2,2-Dichlorcyclopropancarbonsäurechlorid in 100 ml absolutem Tetrahydrofuran 6 Stunden am Rückfluß gehalten. Man saugt bei 10°C ab, wäscht zuerst mit Tetrahydrofuran, dann mit Wasser und kristallisiert aus Dimethylformamid/Wasser um. Man erhält 7,5 g (73% d.Th.) 6-[p-(2,2-Dichlorcyclopropylcarbonylamino)phenyl]-4,5-dihydro-3(2H)-pyridazinon, farblose Kristalle, Schmelzpunkt 267 bis 268°C.

Analyse für $C_{14}H_{13}Cl_2N_3O_2$:
ber.; C 51,6 H 4,0 Cl 21,7 N 12,9%
gef.: C 51,3 H 4,1 Cl 21,6 N 12,9%

Beispiel 11

5,0 g (24,6 mmol) 6-(p-Aminophenyl)-4,5-dihydro-5-methyl-3(2H)-pyridazinon werden unter Rühren in 100 ml absolutem Tetrahydrofuran durch Erwärmen auf 60°C gelöst. Man läßt die Lösung auf Raumtemperatur abkühlen, tropft 5,15 g (29,7 mmol) 2,2-Dichlorcyclopropancarbonsäurechlorid, gelöst in 10 ml absolutem Tetrahydrofuran, zu und rührt noch 6 Stunden bei Rückflußtemperatur nach. Man engt auf ca. 50 ml ein, saugt bei 10°C ab, wäscht mit Wasser und kristallisiert aus Dimethylformamid/Wasser um. Man isoliert 5,0 g (60% d.Th.) 6-[p-(2,2-Dichlorcyclopropylcarbonylamino)phenyl]-4,5-dihydro-5-methyl-3(2H)-pyridazinon, farblose Kristalle, Schmelzpunkt 252 bis 253°C.

Analyse für $C_{15}H_{15}Cl_2N_3O_2$:
ber.: C 53,0 H 4,4 Cl 20,8 N 12,4%
gef.: C 53,0 H 4,4 Cl 20,7 N 12,5%

Beispiel 12

Führt man das Beispiel 1 unter Verwendung von 2,2-Dichlor-1-methylcyclopropancarbonsäurechlorid (8,9 g (47,5 mmol)) an Stelle von Cyclopropancarbonsäurechlorid durch, so erhält man nach der Umkristallisation aus Dimethylformamid/Wasser 4,0 g (37% d.Th.) 6-[p-(2,2-Dichlor-1-methylcyclopropylcarbonylamino)phenyl]-4,5-dihydro-3(2H)-pyridazinon, farblose Kristalle, Schmelzpunkt 209 bis 210°C.

Analyse für $C_{15}H_{15}Cl_2N_3O_2$:
ber.: C 53,0 H, 4,4 Cl 20,8 N 12,4%
gef.: C 52,9 H 4,5 Cl 20,4 N 12,4%

Beispiel 13

4,0 g (21,1 mmol) 6-(p-Aminophenyl)-4,5-dihydro-3(2H)-pyridazinon werden mit 4,3 g (23,4 mmol) trans-2-Bromcyclopropancarbonsäurechlorid in 100 ml absolutem Tetrahydrofuran zuerst 20 Stunden bei Raumtemperatur und dann noch 6 Stunden bei Rückflußtemperatur gehalten. Man saugt bei 10°C ab. wäscht mit Wasser und kristallisiert aus Dimethylformamid/Wasser um. Man isoliert 4,1 g (58% d.Th.) trans-6-[p-(2-Bromcyclopropylcarbonylamino)phenyl]-4,5-dihydro-3(2H)-pyridazinon, farblose Kristalle, Schmelzpunkt 260 bis 261°C (mit Zersetzung).

Analyse für $C_{14}H_{14}BrN_3O_2$:
ber.: C 50,0 H 4,2 Br 23,8 N 12,5%
gef.: C 49,8 H 4,2 Br 24,3 N 12,7%

Beispiel 14

4,0 g (19,7 mmol) 6-(p-Aminophenyl)-4,5-dihydro-5-methyl-3(2H)-pyridazinon werden unter Rühren in 100 ml absolutem Tetrahydrofuran durch Erwärmen auf 60°C gelöst. Man läßt die Lösung auf Raumtemperatur abkühlen, tropft 4,0 g (21,8 mmol) trans-2-Bromcyclopropancarbonsäurechlorid zu und rührt zuerst 20 Stunden bei Raumtemperatur und dann noch 1 Stunde bei Rückflußtemperatur nach. Man engt auf ca. 50 ml ein, gibt Wasser zu und saugt ab. Nach dem Waschen mit Wasser und dem Umkristallisieren aus Dimethylformamid/Wasser erhält man 6,5 g (94% d.Th.) trans-6-[p-(2-Bromcyclopropylcarbonylamino)phenyl]-4,5-dihydro-5-methyl-3(2H)-pyridazinon, farblose Kristalle, Schmelzpunkt 238 bis 239°C (mit Zersetzung).

Analyse für $C_{15}H_{16}BrN_3O_2$:
ber.: C 51,4 H 4,6 Br 22,8 N 12,0%
gef.: C 51,6 H 4,6 Br 22,4 N 11,8%

Beispiel 15

Wird im Beispiel 3 anstatt Cyclopropancarbonsäurechlorid Cyclobutancarbonsäurechlorid (3,9 g (32,9 mmol)) eingesetzt, so erhält man nach der Umkristallisation aus Ethanol/Wasser 2,6 g (31% d.Th.) 6-(p-Cyclobutylcarbonylaminophenyl)-4,5-dihydro-5-methyl-3(2H)-pyridazinon, farblose Kristalle, Schmelzpunkt 163 bis 164°C.

Analyse für $C_{16}H_{19}N_3O_2$:

ber.: C 67,3 H 6,7 N 14,7%

gef.: C 66,9 H 6,6 N 14,8%

Die in der folgenden Tabelle 2 angegebenen Beispiele 16 und 17 werden nach der im Beispiel 3 beschriebenen Methode hergestellt.

TABELLE 2

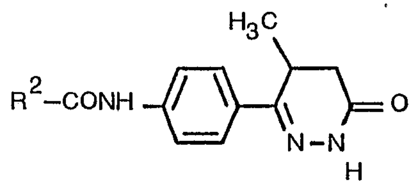

| Beispiel | $R^2$ | Schmp. [°C] | | Analyse (%) C | H | Cl | N |
|---|---|---|---|---|---|---|---|
| 16 | 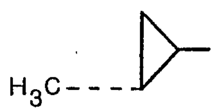 | 258–260 (Dimethylformamid/Wasser) | ber.: | 67,3 | 6,7 | – | 14,7 |
| | | | gef.: | 66,9 | 6,8 | – | 14,7 |
| 17 | 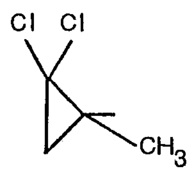 | 219–220 (Dimethylformamid/Wasser) | ber.: | 54.3 | 4,8 | 20,0 | 11,9 |
| | | | gef.: | 54.5 | 4,8 | 19,6 | 12,1 |

Formulierungsbeispiele, die in üblicher Weise hergestellt werden:

1. Tabletten:

| | |
|---|---|
| Wirkstoff | 10 mg |
| Polyvinylpyrrolidon (mittl. M.G. 25 000) | 170 mg |
| Polyäthylenglykol (mittl. M.G. 4 000) | 14 mg |
| Hydroxypropylmethylcellulose | 40 mg |
| Talkum | 4 mg |
| Magnesiumstearat | 2 mg |
| | 240 mg |

Der Wirkstoff wird mit Polyvinylpyrrolidon in 10% iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M.G. 4 000), Hydroxypropylmethylcellulose, Talkum und Magnesiumstearat vermischt und zu Tabletten à 240 mg verpreßt.

2. Beispiel für Dragees:

| | |
|---|---|
| Wirkstoff | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wird mit einer 8 %igen wäßrigen Lösung des Polyvinylpyrrolidons durch Sieb 1,5 mm granuliert, bei 50°C getrocknet und nochmals durch Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

**Patentansprüche**

1. 6-(p-Acylaminophenyl)-4,5-dihydro-3(2H)-pyridazinone der allgemeinen Formel I

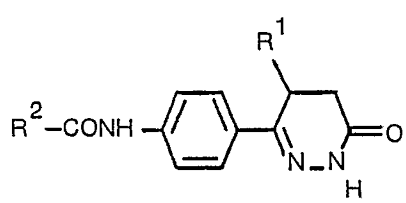

I,

in der $R^1$ für ein Wasserstoffatom oder Methyl steht und $R^2$ einen Cycloalkylrest mit 3 oder 4 C—Atomen im Ring, der gegebenenfalls ein- bis dreifach durch Halogenatome und/oder Methylreste substituiert ist, bedeutet.

2. Verbindungen der Formel I, in denen $R^1$ für ein Wasserstoffatom oder Methyl steht und $R^2$ einen gegebenenfalls ein- bis dreifach durch Halogenatome, insbesondere Chlor- oder Bromatome, und/oder Methylreste substiutierten Cyclopropylrest bedeutet.

3. Verfahren zur Herstellung von Verbindungen der Formel I nach Anspruch 1, dadurch gekennzeichnet, daß man eine Verbindung der Formel II

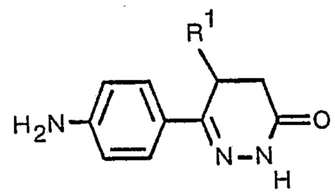

II,

in der $R^1$ die für Formel I angegebenen Bedeutungen hat, mit einem Acylierungsmittel der Formel III

$$R^2COY \qquad III,$$

in der $R^2$ die für Formel I angegebenen Bedeutungen hat und für Y OH, ein Halogenatom, ein niederer Alkoxyrest oder der Rest $OCOR^2$ steht, in an sich bekannter Weise umsetzt oder daß man eine Verbindung der Formel IV

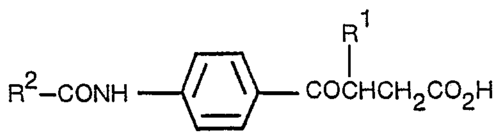

IV,

in der $R^1$ und $R^2$ die für Formel I angegebenen Bedeutungen haben, mit Hydrazin cyclisiert.

4. Therapeutisches Mittel, gekennzeichnet durch einen Gehalt an einer Verbindung der Formel I nach Anspruch 1 als Wirkstoff neben üblichen Trägerstoffen und Verdünnungsmitteln.

5. Eine Verbindung der Formel I nach Anspruch 1 als Arzneimittel bei der Behandlung von hohem Blutdruck oder thrombo-embolischen Erkrankungen.

**Revendications**

1. 6-(p-Acylaminophényl)-4,5-dihydro-3(2H)-pyridazinones de la formule générale I

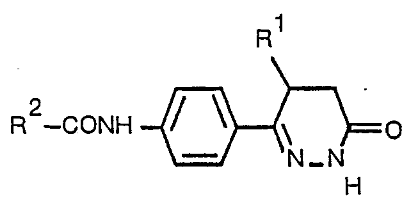

I,

dans laquelle $R^1$ désigne un atome d'hydrogène ou un radical méthyle et $R^2$ un groupe cycloalkyle à noyau en $C_3$ ou $C_4$, éventuellement mono- à tri-substitué par des atomes d'halogène et(ou) des radicaux méthyle.

2. Composés de la formule I, dans laquelle $R^1$ désigne un atome d'hydrogène ou un radical méthyle et $R^2$ un groupe cyclopropyle éventuellement mono- à tri-substitué par des atomes d'halogène, en particulier des atomes de chlore ou de brome, et(ou) des radicaux méthyle.

3. Procédé de préparation de composés de la formule I selon la revendication 1, caractérisé en ce que, soit on fait réagir de manière connue en soi un composé de la formule II

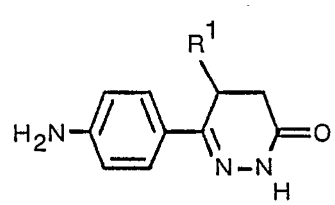

II,

dans laquelle $R^1$ possède les significations définies pour la formule I, avec un agent d'acylation de la formule III

$$R^2COY \qquad (III),$$

dans laquelle $R^2$ possède les significations définies pour la formule I et Y représente un groupe OH, un atome d'halogène, un groupe alcoxy inférieur ou un reste $—OCOR^2$, soit on cyclise avec l'hydrazine un composé de la formule IV

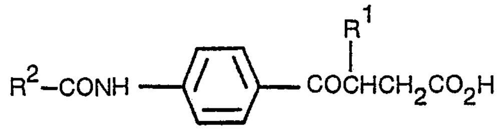

IV,

dans laquelle $R^1$ et $R^2$ possèdent les significations définies pour la formule I.

4. Composition pharmaceutique, contenant comme principe actif un composé de la formule I selon la revendication 1 à côté de matières supports et de diluants usuels.

5. Composé de la formule I selon la revendication 1 comme médicament pour le traitement d'une tension sanguine élevée ou d'affections thrombo-emboliques.

**Claims**

1. A 6-(p-acylaminophenyl)-4,5-dihydro-3(2H)-pyridazinone of the general formula I

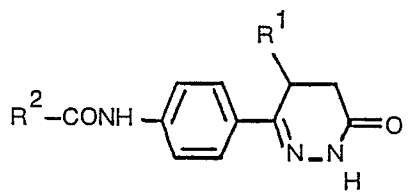

I,

where $R^1$ is hydrogen or methyl, and $R^2$ is cycloalkyl which has 3 or 4 carbon atoms in the ring and is optionally mono-, di- or trisubstituted by halogen and/or methyl.

2. A compound of the formula I, where $R^1$ is hydrogen or methyl, and $R^2$ is cyclopropyl which is optionally mono-, di- or trisubstituted by halogen, especially chlorine or bromine, and/or methyl.

3. A process for the preparation of a compound of the formula I as claimed in claim 1, wherein a compound of the formula II

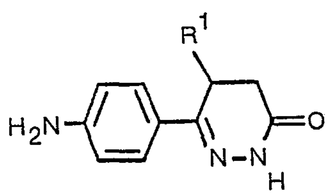

II,

where $R^1$ has the meanings given for formula I, is reacted with an acylating agent of the formula III

$$R^2COY \qquad III,$$

where $R^2$ has the meanings given for formula I and Y is OH, halogen, lower alkoxy or $OCOR^2$, in a conventional manner, or wherein a compound of the formula IV

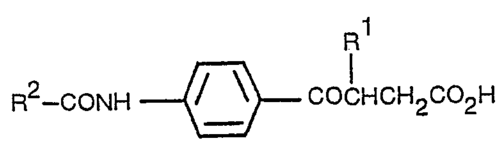

IV,

where $R^1$ and $R^2$ have the meanings given for formula I, is cyclized with hydrazine.

4. A therapeutic agent which contains a compound of the formula I as claimed in claim 1, as the active compound, in addition to conventional carriers and diluents.

5. A compound of the formula I as claimed in claim 1 as a drug for treating high blood pressure or thrombo-embolic disorders.